# EUROPEAN PATENT SPECIFICATION

(11) **EP 1 773 300 B1**
(45) Date of publication and mention of the grant of the patent: **04.09.2013**
(21) Application number: 05759646.2
(22) Date of filing: 12.07.2005
(51) Int. Cl.: A61K 9/16, A61K 9/20, A61K 31/55

(54) **SOLID PHARMACEUTICAL COMPOSITION COMPRISING MIRTAZAPINE**
FESTE PHARMAZEUTISCHE ZUSAMMENSETZUNG MIT MIRTAZAPIN
COMPOSITION PHARMACEUTIQUE SOLIDE COMPRENANT DE LA MIRTAZAPINE

(30) Priority: 13.07.2004 DE 102004034043
(43) Date of publication of application: 18.04.2007
(73) Proprietor: KRKA, tovarna zdravil, d.d., Novo mesto, 8501 Novo mesto (SI)
(72) Inventor: PUNCUH-KOLAR, Alesa, 8220 Smarjeske toplice (SI); TURK, Urska, 8000 Novo mesto (SI); KINCL, Maja, 8000 Novo mesto (SI); RAJER, Tadeja, 8000 Novo mesto (SI); FERLAN, Andrej, 1275 Smartno pri Litiji (SI); GARTNAR, Barbara, 1215 Medvode (SI); CERNOSA, Lidia, 8311 Kostanjevica na Krki (SI)
(74) Representative: UEXKÜLL & STOLBERG
(86) International application number: PCT/EP2005/007552
(87) International publication number: WO 2006/005578

(56) References cited:
- EP-A- 0 436 252
- WO-A-01/26621
- WO-A-01/74340
- WO-A-03/103629
- ARTHUR H. KIBBE: "Handbook of pharmaceutical excipients, 3rd edition" 2000, PHARMACEUTICAL PRESS , XP002366013 page 244 - page 248
- ARTHUR H. KIBBE: "Handbook of pharmaceutical excipients, 3rd edition", 2000, PHARMACEUTICAL PRESS * page 102 - page 103 *
- 'US Pharmacopeia 24', * page 2254 *
- CHAMBIN O ET AL: "Effects of different cellulose derivatives on drug release mechanism studied at a preformulation stage", JOURNAL OF CONTROLLED RELEASE, ELSEVIER, AMSTERDAM, NL, vol. 95, no. 1, 20 February 2004 (2004-02-20), pages 101-108, XP004487819, ISSN: 0168-3659, DOI: 10.1016/J.JCONREL.2003.11.009
- 'Derivative (chemistry)', [Online] 20 November 2011, Wikipedia Retrieved from the Internet: <URL:http://en.wikipedia.org/wiki/Derivativ e_(chemistry)>

## Description

The present invention relates to a solid pharmaceutical composition for oral administration which comprises 2-methyl-1,2,3,4,10,14β-hexahydrobenzo[*c*]pyrazino[1,2a]pyrido-[3,2*f*]-azepine, in the following referred to by its generic name "mirtazapine" and in particular those which are in form of orally-disintegrating tablets.

Mirtazapine is known as an anti-depressant, recognized as a noradrenergic and specific serotonergic antidepressant (NaSSA) that enhances both noradrenergic and serotonergic neurotransmission by means of antagonism at presynaptic α₂-adrenoreceptors while blocking postsynaptic serotonin 5-HT₂ and 5-HT₃ receptors. It is represented by the following structural formula:

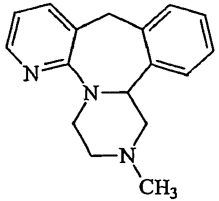

In the field of pharmacy, there have been in the past attempts to provide a type of pharmaceutical composition for oral administration which results in improved patient compliance in comparison to conventional solid dosage forms such as capsules and tablets. In particular paediatric and geriatric patients often experience difficulties in swallowing solid dosage forms. Besides, conventional solid dosage forms are not suitable in case the patient has no easy access to water. Thus, orally-disintegrating compositions represent an alternative for such patients.

A satisfactory orally disintegrating dosage form needs to meet a number of requirements. Firstly, it has to disintegrate in the mouth spontaneously. However, the active ingredient should not be released from the dosage form in the oral cavity as is the case for sublingual or buccal formulations. Otherwise the active ingredient would be absorbed already in the mouth leading to a different distribution and metabolization of the ingredient in the body compared to a conventional tablet which releases the active ingredient in the gastro-intestinal tract. The consequence would be a difference in relation to bioavailability which would cause significant problems.

Moreover, a premature release in the mouth could also lead to problems due to the often unpleasant taste of the active ingredient.

Moreover, mirtazapine shows a local anaesthetic effect, which is found particularly annoying by patients experiencing release of mirtazapine in the oral cavity. This enhances the necessity to prevent oral release of this drug from a corresponding oral formulation.

To fulfil all these requirements the formulation for a specific drug needs to be adapted in particular by a careful selection of the excipients used. However, the excipients may cause a problem as they may lead to formulations which are not bioequivalent to the corresponding conventional dosage forms.

Orally disintegrating pharmaceutical compositions are known.

WO 01/26621 discloses orally-disintegrating tablets which comprise mirtazapine applied in form of a layer on an inert core. The tablets also comprise a polymeric layer which should prevent the oral release of the drug. The polymeric layer is made of hydroxypropyl cellulose, hydroxpropylmethyl cellulose or acrylates, like Eudragit^{®} polymers. However, the use of inert cores makes the manufacturing process difficult and further the presence of in particular Eudargit^{®} polymers has the disadvantage of uneven surfaces due to its agglomeration tendency. Moreover, the presence of an inert core may lead to segregation problems when compressing the coated particles to produce tablets. Furthermore, the use of Eudragit^{®} requires a very careful control of the temperature during the layering process.

EP 0 436 252 B1 discloses granules which comprise mirtazapine in a sparingly soluble form, such as the base, a cellulose derivative which is soluble in water and organic solvents, such as hydroxypropyl cellulose and hydroxypropylmethyl cellulose, as well as a polymer which is insoluble in water, namely ethylene vinylacetate polymer, polyethyleneglycol ester and in particular a polyacrylate, like Eudargit® RS. However, the granules obtained still do not satifactorily prevent the release of mirtazapine and the associated unpleasant taste and anaesthetic effect in the oral cavity.

Thus, there is a need for a pharmaceutical composition of mirtazapine which avoids the above mentioned problems and in particular disintegrates quickly in the oral cavity without releasing substantial amounts of the active ingredient and hence avoids the occurrence of the unpleasant taste of mirtazapine. Moreover, such a composition should not require the use of expensive excipients, such as inert cores, and should be obtainable by a simple and quick process.

These objects are surprisingly solved by the solid pharmaceutical composition according to claims 1 to 14. The invention also relates to the process according to claim 15.

The solid pharmaceutical composition for oral administration according to the invention comprises
a) mirtazapine or a salt or solvate thereof,
b) at least one cellulose derivative which is insoluble in water and which is selected from ethyl cellulose or methyl cellulose, and
c) optionally at least one other excipient.

The component (a) of the composition is mirtazapine. The mirtazapine can also be present in form of a salt thereof. Examples of useful salts are the addition salts with an inorganic acid selected from hydrochloric, hydrobromic, phosphoric, nitric and sulphuric acid or with an organic acid selected from acetic, propanoic, hydroxyacetic, oxalic, lactic, pyruvic, malonic, succinic, fumaric, malic, glutaric, tartaric, maleic, citric, cyclamic, sulphonic, benzensulphonic, pamoic and the like.

Examples of useful salts are also described in DE 26 14 406.

In addition, the mirtazapine can also be used in form of different polymorphic or pseudopolymorphic forms, such as a solvate. It can be a solvate with water or organic solvents as disclosed in DE 26 14 406. It is also possible to use the mirtazapine in anhydrous form as disclosed in EP 1 225 174 and EP 1 209 152 or in micronized form as disclosed in US 2002/0072602.

Mirtazapine can be also used in form of the (R)- or (S)-enantiomer in substantially pure form, i.e. having an enantiomeric purity of greater than 95% and preferably greater than 99 %, as well as in form of mixtures of the enantiomers in any proportion including a racemic mixture.

It is preferred that the composition comprises from 1 to 95 %, more preferably 3 to 75% and most preferably 5 to 50 % by weight of mirtazapine or a salt or solvate thereof. For granules a particularly preferred range is from 24 to 32 % by weight, and for tablets 7.4 to 11.4 % by weight.

It is also preferred that a single dosage form of the pharmaceutical composition according to the invention, such as a granulate or tablet or capsule, comprises from 1 mg to 90 mg mirtazapine or salt or solvate thereof, calculated as mirtazapine.

The component (b) of the composition is at least one cellulose derivative which is insoluble in water. A cellulose derivative is considered insoluble in water if 1 part by weight of cellulose derivative is soluble in more than 10,000 parts by weight of solvent at a temperature of 20°C.

The composition preferably comprises 0.01 to 25.0 % by weight, more preferably 0.02 to 15 % by weight and most preferably 0.03 to 10 % by weight of the cellulose derivative (b). A particularly preferred range for granules is from 1 to 3 % by weight, and for tablets from 0.4 to 0.9 % by weight.

The cellulose derivative (b) is selected from ethyl cellulose and methyl cellulose. It was surprisingly found out that, even though these derivatives are conventionally used as sustained release agents, their inclusion does not delay the release of mirtazapine at the acidic pH prevailing in the gastro-intestinal tract, but prevent the release of mirtazapine in the oral cavity. Further, the disintegration and dissolution behaviour of these materials are substantially less pH dependent than the Eudragit® materials used according to the prior art.

In a preferred embodiment of the invention, the composition does not comprise polyacrylates or polymethacrylates, which are commonly sold under the brand Eudragit®. It is surprising that despite the absence of such materials in this embodiment the desired properties are achieved.

It is further preferred that at least a part, preferably virtually all, of the component (a) is in contact with the cellulose derivative (b), in particular is embedded in the cellulose derivative (b). This facilitates the accomplishing of the desired prevention of oral release of the active ingredient (a).

The weight ratio of component (a) to component (b) is preferably greater than 3 to 1, in particular greater than 10:1.

The composition can also comprise at least one other excipient (c). A preferred excipient is a filler.

It is preferred that the composition comprises 10 to 90 % by weight and in particular 40 to 90 % by weight of filler.

Suitable fillers include lactose, sucrose, starch, calcium phosphate, calcium sulfate, microcrystalline cellulose, dextrates, dextrose, fructose, silicified microcrystalline cellulose. Mixtures of different fillers can be also used. The preferred filler is lactose, which can be in anhydrous or hydrous form, for example lactose monohydrate. The average particle size of the lactose usually ranges from 5 to 1,000 µm

The composition according to the invention is preferably in form of granules.

In a preferred embodiment, these granules consist essentially of component (a), e.g. mirtazapine, the cellulose derivative (b) and filler. Thus, such embodiment very much limits the number of the necessary excipients and is very easy to prepare.

It has surprisingly been found out that by using mirtazapine in combination with the water insoluble cellulose derivative (b) granules can be prepared which do not require an additional coating to prevent mirtazapine from being released orally. This is very advantageous since the application of an additional coating requires an additional process step and is time-consuming.

Furthermore, this simple combination unexpectedly results in the release of mirtazapine being sufficiently prevented in the oral cavity. The superiority of the prevention of release of mirtazapine in comparision to formulations according to the prior art is shown by the in vitro drug release test given in Example 3. The results of this test are also represented in Table 1 showing that at a pH prevailing in the oral cavity the release of mirtazapine from the granulate according to the invention was substantially lower than for a granulate prepared according to EP 0 436 252. Nevertheless, the desired immediate release of mirtazapine at the acidic pH of the gastric juice is achieved with the granulate according to the invention.

The granules can simply be prepared by mixing component (a) and optional filler, and spraying a solution of the cellulose derivative (b) on the mixture obtained, followed by drying and sieving to result in granules.

The solution of the cellulose derivative can be prepared by using solvents conventionally employed in pharmacy. The solvent can be water, an organic solvent or a mixture thereof. It is preferred to use a mixture of ethanol and water, and in particular ethanol is used as solvent.

It is very advantageous that the process can be carried out as a one-step process using conventional equipment, such as a high shear mixer (granulator) or any other conventional equipment, such as a fluid bed granulator. The granules according to the invention can be used as such or incorporated in different dosage forms, like tablets, capsules or orally disintegrating tablets. Preferably, the granules are used to prepare orally disintegrating tablets.

Thus, in a further preferred embodiment, the composition according to the invention takes the form of an orally-disintegrating tablet.

It is preferred that such a tablet further comprises sugar alcohol, disintegrant or combinations thereof.

Preferred sugar alcohols are sorbitol and mannitol.

Useful sugar alcohols and mixtures thereof are disclosed in WO 03/051338. These materials are also available under the trade name Pharmaburst, for example Pharmaburst C1, Pharmaburst B1 and Pharmaburst B2 from SPI Pharma. Pharmaburst comprises spray-dried mannitol and sorbitol and can additionally comprise a disintegrant, such as crospovidone.

It is preferred that the orally-disintegrating tablet comprises 1 to 50 %, more preferably 1 to 40 % and most preferably 2 to 25 % of a disintegrant. The disintegrant can be selected from crospovidone, low-substituted hydroxypropyl cellulose, calcium silicate, sodium starch glycolate or croscarmellose sodium or a combination thereof, with crospovidone being particularly preferred.

Particularly preferred are tablets which comprise combinations of spray-dried mannitol and sorbitol with crospovidone.

The invention also relates to a process for preparing the tablets comprising
(i) granulating mirtazapine, a salt or solvate thereof with a solution of the cellulose derivative (b),
(ii) optionally adding to the granules obtained sugar alcohol, disintegrant or a combination therof, and
(iii) compressing the granules of (i) or the mixture of (ii) to obtain tablets.

An inert gas atmosphere can be used in this process. Moreover, it is possible to effect the packaging in an inert gas atmosphere, preferably a nitrogen atmosphere.

It has surprisingly been found that by selecting the above components an orally-disintegrating tablet can be prepared by using a very simple and fast tableting process. These tablets do not require use of effervescent agents and disintegrate in less than 40 seconds, preferably less than 30 seconds.

At the same time mirtazapine is rapidly released in an acid environment and absorbed only trough the gastrointestinal route.

The composition according to the invention, and in particular the tablet, can further comprise auxiliary agents such as lubricants, sweeteners, and flavouring agents.

Examples of suitable lubricants are magnesium stearate, magnesium lauryl sulfate, sodium stearyl fumarate, hydrogenated vegetable oil, fatty acid, polyethylene glycol and stearic acid.

Examples of sweeteners are acesulfame K, sucralose, alitame, aspartame, saccharin sodium, dipotassium glycyrrhizinate, stevia and thaumatin.

Flavouring agents can be natural or synthetic materials, such as orange flavour, spearmint flavour and the like.

The invention is in the following further illustrated by reference to following examples.

### Examples

### Example 1 - Preparation of granules of mirtazapine and cellulose derivative

| | |
|---|---|
| Mirtazapine | 30 mg |
| Lactose | 75 mg |
| Ethyl cellulose | 2.16 mg |

Mirtazapine and lactose in the above given amounts were blended for 3 minutes in a high shear mixer. Ethyl cellulose was dissolved in 14.35 mL of ethanol (96% m/V) and this solution was sprayed onto the powder mixture. The obtained moistened mixture was further kneaded for 1.5 minutes. The kneaded mixture was then dried at 50°C in a fluid bed drier and sieved to give a granules having a particle size of less than 1000 µm.

### Example 2 - Preparation of granules (Reference example)

| | |
|---|---|
| mirtazapine | 24.0 g |
| Eudragit RS | 80.0 g |
| Hydroxypropyl cellulose | 8.0 g |
| mannitol | 888.0 g |
| total | 1000.0 g |

For comparative purposes the Example 2 of EP 0 436 252 was repeated. Thus, 80.0 g Eudragit RS, 8.0 g of hydroxypropyl cellulose and 24 g of mirtazapine were dissolved successively in 125 milliliters of a mixture of ethanol and acetone (1:1 V/V). This solution was applied on 888.0 g of mannitol and the obtained mixture was kneaded for 3 minutes in a mixer. The resulting mass is sieved through a 2000 µm sieve and dried for 3 hours at 50°C under vacuum to give granules.

### Example 3 - Comparision of release profiles of example 1 and 2

In this example the release profile of mirtazapine of the granulate according to example 2 was compared with that of the granulate according to the invention of example 1.

For this purpose an in vitro dissolution test was carried out utilizing the United States Pharmacopeia dissolution test, Apparatus 2, paddle method, at 50 rpm in 500 mL of phosphate buffer at a pH of 7.2, which simulates the pH in the oral cavity.

It can be seen from the Table I below that after 1 minute 11 % by weight of mirtazapine is released from the granulate of Example 2 (Reference), while from the granulate of Example 1 (Invention) only 2 % by weight of mirtazapine were released.

Thus, the undesired release of mirtazapine from granules according to the invention is much more satisfactorily prevented than from granules acording to the prior art.

**Table I**

| Time (min) | Average % of released mirtazapine by *in vitro* dissolution test at pH 7.2 | |
|---|---|---|
| | Granulate from Example 1 | Granulate from Example 2 |
| 0 | 0 | 0 |
| 1 | 2 | 11 |
| 2 | 3 | 22 |
| 3 | 3 | 32 |
| 4 | 4 | 39 |
| 5 | 4 | 43 |
| 10 | 6 | 55 |
| 15 | 7 | 60 |

### Example 4 - Orally-disintegrating tablets

| | |
|---|---|
| Granules of Example 1 | 107.16 mg |
| Pharmaburst^{®} C1 | 195.94 mg |
| Orange flavour | 6.4 mg |
| Aspartame | 3.2 mg |
| Sodium stearyl fumarate | 6.4 mg |

Granules of Example 1 were mixed with the above given amounts of Pharmaburst^{®} C1, orange flavour, aspartame and sodium stearyl fumarate in a biconic mixer. The obtained mixture was compressed into tablets using a force of less than 60 N. The so-obtained tablets displayed a disintegration time in the range of 40 to 20 s.

The disintegration time was measured in purified water at 37°C according to the method described in Ph. Eur. Chapter 2.9.1. Test A, page 4683, supplement 4.8 07/2004.

## Claims

1. Solid pharmaceutical composition for oral administration which comprises
(a) mirtazapine or a salt or solvate thereof,
(b) at least one cellulose derivative which is insoluble in water and which is selected from ethyl cellulose or methyl cellulose, and
(c) optionally at least one other excipient
wherein a cellulose derivative is considered insoluble in water if 1 part by weight of the cellulose derivative requires more than 10,000 parts by weight of water to dissolve at a temperature of 20°C.

2. Composition according to claim 1, which comprises 0.01 to 25.0 % by weight of the cellulose derivative (b).

3. Composition according to claim 1 or 2, wherein at least a part of the component (a) is in contact with the cellulose derivative (b).

4. Composition according to any one of claims 1 to 3, wherein the other excipient (c) is a filler.

5. Composition according to claim 4, wherein the filler is lactose.

6. Composition according to claim 4 or 5, which comprises 10 to 90 % by weight of filler.

7. Composition according to any one of claims 1 to 6, which does not comprise polyacrylates or polymethacrylates.

8. Composition according to any one of claims 1 to 7, which is in form of granules.

9. Composition according to any one of claims 1 to 7, which is in form of an orally-disintegrating tablet.

10. Composition according to claim 9, wherein the tablet further comprises sugar alcohol, disintegrant or a combination thereof.

11. Composition according to claim 9 or 10, wherein the sugar alcohol is selected from mannitol and sorbitol.

12. Composition according to any one of claims 9 to 11, wherein the disintegrant is crospovidone.

13. Composition according to any one of claims 9 to 12, which comprises 1 to 50 % by weight of disintegrant.

14. Composition according to any one of claims 1 to 13, wherein the cellulose derivative (b) prevents release of component (a) in the oral cavity.

15. Process for preparing the composition according to any one of claims 9 to 14 comprising
(i) granulating mirtazapine, a salt or solvate thereof with a solution of the cellulose derivative (b),
(ii) addding to the granules obtained sugar alcohol, disintegrant or a combination thereof, and
(iii) compressing the granules to obtain tablets.

## Patentansprüche

1. Feste pharmazeutische Zusammensetzung zur oralen Verabreichung, die
(a) Mirtazapin oder ein Salz oder Solvat davon,
(b) mindestens ein Cellulosederivat, das unlöslich in Wasser ist und ausgewählt ist aus Ethylcellulose oder Methylcellulose, und
(c) gegebenenfalls mindestens einen weiteren Hilfsstoff
enthält, wobei ein Cellulosederivat als unlöslich in Wasser angesehen wird, wenn 1 Gewichtsteil des Cellulosederivats mehr als 10.000 Gewichtsteile Wasser erfordert, um bei einer Temperatur von 20°C gelöst zu werden.

2. Zusammensetzung nach Anspruch 1, die 0,01 bis 25,0 Gew.-% des Cellulosederivats (b) enthält.

3. Zusammensetzung nach Anspruch 1 oder 2, bei der zumindest ein Teil der Komponente (a) in Kontakt mit dem Cellulosederivat (b) vorliegt.

4. Zusammensetzung nach einem der Ansprüche 1 bis 3, bei der der weitere Hilfsstoff (c) ein Füllstoff ist.

5. Zusammensetzung nach Anspruch 4, bei der der Füllstoff Lactose ist.

6. Zusammensetzung nach Anspruch 4 oder 5, die 10 bis 90 Gew.-% Füllstoff enthält.

7. Zusammensetzung nach einem der Ansprüche 1 bis 6, die keine Polyacrylate oder Polymethacrylate enthält.

8. Zusammensetzung nach einem der Ansprüche 1 bis 7, die in Form von Granulatkörnern vorliegt.

9. Zusammensetzung nach einem der Ansprüche 1 bis 7, die in Form einer oral zerfallenden Tablette vorliegt.

10. Zusammensetzung nach Anspruch 9, bei der die Tablette weiterhin Zuckeralkohol, Sprengmittel oder eine Kombination davon enthält.

11. Zusammensetzung nach Anspruch 9 oder 10, bei der der Zuckeralkohol ausgewählt ist aus Mannit und Sorbit.

12. Zusammensetzung nach einem der Ansprüche 9 bis 11, bei der das Sprengmittel Crospovidon ist.

13. Zusammensetzung nach einem der Ansprüche 9 bis 12, die 1 bis 50 Gew.-% Sprengmittel enthält.

14. Zusammensetzung nach einem der Ansprüche 1 bis 13, bei der das Cellulosederivat (b) die Freisetzung der Komponente (a) in der Mundhöhle verhindert.

15. Verfahren zur Herstellung der Zusammensetzung gemäß einem der Ansprüche 9 bis 14, bei dem
(i) Mirtazapin, ein Salz oder Solvat davon mit einer Lösung des Cellulosederivats (b) granuliert wird,
(ii) Zuckeralkohol, Sprengmittel oder eine Kombination davon zu den erhaltenen Granulatkörnern gegeben werden und
(iii) die Granulatkörner komprimiert werden, um Tabletten zu erhalten.

## Revendications

1. Composition pharmaceutique solide pour administration par voie orale, qui comprend :
a) de la mirtazapine ou de l'un de ses sels ou de ses solvats,
b) au moins un dérivé de cellulose, insoluble dans l'eau et choisi parmi de l'éthyl-cellulose et de la méthyl-cellulose,
c) et en option, au moins un autre excipient,
étant entendu qu'un dérivé de cellulose est considéré comme étant insoluble dans l'eau s'il faut plus de 10 000 parties en poids d'eau pour dissoudre 1 partie en poids de ce dérivé de cellulose à la température de 20 °C.

2. Composition conforme à la revendication 1, qui comprend de 0,01 à 25,0 % en poids du dérivé de cellulose (b).

3. Composition conforme à la revendication 1 ou 2, dans laquelle au moins une partie du composant (a) est en contact avec le dérivé de cellulose (b).

4. Composition conforme à l'une des revendications 1 à 3, dans laquelle l'autre excipient (c) est un excipient de charge.

5. Composition conforme à la revendication 4, dans laquelle l'excipient de charge est du lactose.

6. Composition conforme à la revendication 4 ou 5, qui comprend de 10 à 90 % en poids de l'excipient de charge.

7. Composition conforme à l'une des revendications 1 à 6, qui ne comprend ni polyacrylates ni polyméthacrylates.

8. Composition conforme à l'une des revendications 1 à 7, qui se présente sous forme de granules.

9. Composition conforme à l'une des revendications 1 à 7, qui se présente sous forme de comprimé se désagrégeant dans la bouche.

10. Composition conforme à la revendication 9, dans laquelle le comprimé comprend en outre un alcool de sucre, un agent de désagrégation ou une combinaison de tels adjuvants.

11. Composition conforme à la revendication 9 ou 10, pour laquelle l'alcool de sucre est choisi parmi du mannitol et du sorbitol.

12. Composition conforme à l'une des revendications 9 à 11, dans laquelle l'agent de désagrégation est de la crospovidone.

13. Composition conforme à l'une des revendications 9 à 12, qui comprend de 1 à 50 % en poids de l'agent de désagrégation.

14. Composition conforme à l'une des revendications 1 à 13, dans laquelle le dérivé de cellulose (b) empêche la libération du composant (a) dans la cavité buccale.

15. Procédé de préparation d'une composition conforme à l'une des revendications 9 à 14, comportant les étapes suivantes :
i) mettre de la mirtazapine, ou de l'un de ses sels ou de ses solvats, sous la forme de granules, avec une solution du dérivé de cellulose (b),
ii) ajouter aux granules ainsi obtenus un alcool de sucre, un agent de désagrégation ou une combinaison de tels adjuvants,
iii) et compresser ces granules pour en faire des comprimés.
